# EUROPÄISCHE PATENTSCHRIFT

(11) **EP 0 972 017 B1**
(45) Veröffentlichungstag und Bekanntmachung des Hinweises auf die Patenterteilung: **06.10.2004**
(21) Anmeldenummer: 98907950.4
(22) Anmeldetag: 16.01.1998
(51) Int. Cl.: C12N 15/00, A01K 67/027, C12N 9/64

(54) **EFFIZIENTER KERNTRANSFER MIT FETALEN FIBROBLASTEN**
EFFICIENT NUCLEAR TRANSFER USING FETAL FIBROBLASTS
TRANSFERT DE NOYAU EFFICACE AVEC DES FIBROBLASTES FOETAUX

(43) Veröffentlichungstag der Anmeldung: 19.01.2000
(73) Patentinhaber: Agrobiogen GmbH, Larezhausen, 86567 Hilgertshausen (DE)
(72) Erfinder: BREM, Gottfried, Agrobiogen GmbH, Larezhausen, D-86567 Hilgertshausen (DE); DURCOVA-HILLS, Gabriela, Hackerstrasse 27, 85764 Oberschleissheim (DE); MÜLLER, Sigrid, Joseph Baumann Gasse 1, 1210 Wien (AT); SCHERNTHANER, Wolfgang, Hackerstrasse 27, 85764 Oberschleissheim (DE); WENIGERKIND, Hendrik, Joseph Baumann Gasse 1, 1210 Wien (AT); WOLF, Eckhard, Joseph Baumann Gasse 1, A-1210 Wien (AT); ZAKHARTCHENKO, Valeri, Hackerstrasse 27, 85764 Oberschleissheim (DE)
(74) Vertreter: Becker Kurig Straus
(86) Internationale Anmeldenummer: PCT/EP1998/000230
(87) Internationale Veröffentlichungsnummer: WO 1999/036510

(56) Entgegenhaltungen:
- WO-A-97/07669
- WO-A-98/30683
- WO-A-98/37183
- WO-A-99/01163
- SCHNIEKE, A.E. ET AL.: "Human Factor IX transgenic sheep produced by transfer of nuclei from transfected fetal fibroblasts" SCIENCE., Bd. 278, Nr. 5346, 19. Dezember 1997, Seiten 2130-2133, XP002067036 LANCASTER, PA US
- WILMUT, I. ET AL.: "Viable offspring derived from fetal and adult mammalian cells" NATURE., Bd. 385, Nr. 6619, 27. Februar 1997, Seiten 810-813, XP002067035 LONDON GB
- CHESNE P ET AL: "NUCLEAR TRANSFER IN CATTLE: BIRTH OF CLONED CALVES AND ESTIMATION OF BLASTOMERE TOTIPOTENCY IN MORULAE USED AS A SOURCE OF NUCLEI" LIFE SCIENCES, Bd. 316, 1993, Seiten 487-491, XP000197855
- CAMPBELL K H S ET AL: "NUCLEAR-CYTOPLASMIC INTERACTIONS DURING THE FIRST CELL CYCLE OF NUCLEAR TRANSFER RECONSTRUCTED BOVINE ENBRYOS: IMPLICATIONS FOR DEOXYRIBONUCLEIC ACID REPLICATION AND DEVELOPMENT" BIOLOGY OF REPRODUCTION, Bd. 49, Nr. 5, November 1993, Seiten 933-942, XP000604579
- GADBOIS, D.M. ET AL.: "Multiple kinase arrest points in the G1 phase of nontransformed mammalian cells are absent in transformed cells" PROCEEDINGS OF THE NATIONAL ACADEMY OF SCIENCES OF USA., Bd. 89, September 1992, Seiten 8626-8630, XP002079365 WASHINGTON US
- HEYMAN Y ET AL: "CLONING OF DOMESTIC SPECIES" ANIMAL REPRODUCTION SCIENCE, Bd. 42, Nr. 1/04, 1996, Seiten 427-436, XP000671884
- OTAEGUI P J ET AL: "TRANSFER OF NUCLEI FROM 8-CELL STAGE MOUSE EMBRYOS FOLLOWING USE OFNOCODAZOLE TO CONTROL THE CELL CYCLE" MOLECULAR REPRODUCTION AND DEVELOPMENT, Bd. 39, Nr. 2, Oktober 1994, Seiten 147-152, XP000604559
- CIBELLI, J.B. ET AL.: "Cloned transgenic calves produced from non quiescent fetal fibroblasts" SCIENCE., Bd. 280, Nr. 5367, 22. Mai 1998, Seiten 1256-1258, XP002079366 LANCASTER, PA US
- RITCHIE W.A. ET AL: 'Intracytoplasmic nuclear injection as an alternative to cell fusion for the production of bovine embryos by nuclear transfer.' JOURNAL OF REPRODUCTION AND FERTILITY Bd. 5, 1995, Seite 60

## Beschreibung

Die vorliegende Erfindung betrifft ein Verfahren zur Herstellung von Ungulatenembryonen.

Tiere, insbesondere Nutztiere, werden vom Menschen seit langem für die verschiedensten Zwecke weitergezüchtet und im Hinblick auf bestimmte Eigenschaften fortentwickelt. So wurden beispielsweise Kühe und Stiere mit hohem Zuchtwert für Milchleistung weiterverpaart, um Tiere mit hohem Milchertrag zu erhalten.

In den letzten Jahren gerieten Tiere, insbesondere Vertreter der Ungulaten, wie Schafe, Rinder bzw. Kühe auch als Produktionsstätten ernährungsphysiologisch bzw. pharmazeutisch bedeutender Stoffe in den Mittelpunkt des Forschungsinteresses, da es mit der Entwicklung der Gentechnologie möglich wurde gezielt Tiere herzustellen, denen eine neue Eigenschaft, beispielsweise die Fähigkeit zur Produktion eines bestimmten Arzneimittels, verliehen werden konnte. Das Problem bei der wirtschaftlichen Nutzung derartiger Tiere besteht jedoch darin, daß das ihnen transferierte Genkonstrukt integriert und stabil an die Nachkommen weitergegeben wird.

Zu diesem Zweck wurde versucht, das Problem des Gentransfers dadurch zu lösen, daß dieser in Zellen vorgenommen wird, wobei aus diesen Zellen mittels Klonierung wieder Tiere generiert werden.

In der Fachwelt wird der Begriff des "Klonierens" allgemeinen als Vervielfältigung eines genetischen Materials, abgeleitet von einer einzigen Zelle definiert, was übertragen auf die Embryologie als Erstellung von Embryonen bzw. Tieren mit identischem Genotyp verstanden werden kann. In der Embryologie wird der Keimling während der Blastogenese, also bis zur Entwicklung der Anlage von Primitivorganen als Embryo, in den nachfolgenden Entwicklungsstufen als Fetus bezeichnet. Embryonale Phasen verlaufen je nach Spezies in unterschiedlich langen Zeiträumen ab, so beispielsweise beim Rind in einem Zeitraum von etwa 4 Wochen; wobei bei anderen Spezies innerhalb der Ungulaten kürzere oder auch längere Zeiträume dafür erforderlich sein können.

Zur Klonierung von Tieren, also zur Vervielfältigung eines einem bestimmten Tier eigenen Genotyps wurden bis dato mehrere Wege beschritten.

Einerseits wurden frühe Embryonalstadien und Fortentwicklungen einem mikrochirurgischen Eingriff unterworfen und die jeweils daraus isolierten Teile wurden in vitro bzw. in vivo weitergezüchtet.

Weiter wurde eine als "Chimäres Klonen" bezeichnete mikromanipulatorische Kombination asynchroner Entwicklungsstadien durchgeführt, bei der Blastomere(n) aus Embryonen weiter fortgeschrittener Stadien mit Blastomeren aus früheren Stadien zusammengebracht wurden, mit dem Ziel, die erstgenannten in ihrer Weiterentwicklungskapazität zu unterstützen und somit identische Viellinge zu erzeugen. Die größte Anzahl damit erhaltener Klone lag jedoch lediglich in einer Größenordnung von maximal 5-8.

Eine weitere Vorgehensweise war die parthenogenetische Aktivierung oder die Verpaarung homozygoter Elterntiere, um hinsichtlich bestimmter Eigenschaften Klone zu erhalten.

Da sich die oben genannten Verfahren hinsichtlich Ihrer Effektivität und Zuverlässigkeit jedoch als relativ schlecht erwiesen, wurde ein weiteres Verfahren entwickelt, das generell als Kerntransfer bezeichnet wird.

Dabei werden Zellkerne, die von mehrzelligen Embryonen stammen, in entsprechend vorbereitete Eizellen überführt, wobei genetisch identische Embryonen erstellt werden konnten.

Um eine Klonierung mittels Kerntransfer erfolgreich durchführen zu können, müssen jedoch einige unabdingbare Parameter berücksichtigt werden.

Die Eizelle, die als Empfängerzelle eingesetzt wird, muß das Metaphase-Stadium in der 2. Reifeteilung (Metaphase II) vollendet haben und soll keine eigene nukleare DNA mehr enthalten, d.h. sie soll als sogenannte enukleierte Eizelle vorliegen. Des weiteren sollte das Cytoplasma der Eizelle so wenig wie möglich beeinflußt werden, da die in dem Cytoplasma selbst enthaltenen Stoffe für die Weiterentwicklung, beispielsweise die Teilung der Zelle, von Bedeutung sein können.

Weiterhin muß die nukleare DNA des übertragenen Kerns reprogrammiert werden. Da der (Spender-) Kern von einem mehrzelligen Embryo stammt, hat die jeweilige Spenderzelle bereits einige Teilungszyklen durchlaufen. Dies bedeutet, daß sich die Zelle in einem gegenüber einer totipotenten befruchteten Eizelle fortgeschrittenen Entwicklungsstadium befindet, in dem möglicherweise bereits bestimmte, für die frühe Entwicklung erforderliche Gene abgeschaltet sind.

Aus diesem Grund muß die verwendete Kern-DNA derart reprogrammiert werden, daß die vollständige genetische Information der Kern-DNA wieder zur Verfügung steht und das Teilungsschema des Embryos wieder beim Stadium der Zygote beginnt. Je besser somit diese Reprogrammierung bzw. Aktivierung erreicht werden kann, desto höher liegt die Wahrscheinlichkeit einer erfolgreichen Klonierung, mit der dann auch ein fertig entwickeltes, d.h. lebend geborenes geklontes Tier erhalten werden kann.

Neben der Kern-DNA ist u.a. auch die in dem Cytoplasma vorhandene mRNA von Bedeutung, da diese im Zeitpunkt der Vereinigung von Eizelle und Spenderzelle die für das gegenwärtige Entwicklungs- bzw. Differenzierungsstadium der Spenderzelle erforderlichen Botschaften darstellt und die damit hergestellten Proteine einen Einfluß auf die weitere Entwicklung der Zelle nehmen können.

Das Verfahren des Kerntransfers wurde bereits mit bescheidenem Erfolg eingesetzt. So berichteten Willadsen et al. (Nature **320** (1986), 63-65) über die Klonierung von Lämmern, wobei die Kerne aus Kern-Spenderzellen aus dem 8-Zell-Stadium stammten. Robl et al. (J. Anim. Sci. **64** (1987), 642-647) berichteten über die ersten Kerntransferexperimente beim Rind, wobei ausschließlich mit ex vivo gewonnenen Rinderembryonen als Kernspender gearbeitet wurde. Bei diesen Versuchen war immer eine in vivo Zwischenkultur in Schafeileitern erforderlich. In den folgenden Jahren konnte auch gezeigt werden, daß das Embryonalklonen beim Rind rein in vitro, also unter Verwendung von in vitro produzierten Embryonen und in vitro gereiften Eizellen, erfolgreich durchgeführt werden kann (Sims et al., Proc. Natl. Acad. Sci. USA **91** (1991), 6143-6147).

In der WO 97/07668 wird weiter ein Verfahren zur Wiederherstellung eines tierischen Embryos beschrieben, bei dem generell ein Kern mit einem diploiden Chromosomensatz in eine enukleierte Eizelle überführt wird, die in dem Metaphasenstadium II gehalten wird, wobei die Eizelle beim Einbringen des Kerns erst nach einer gewissen Zeit aktiviert wird. Durch später erfolgende Aktivierung der Eizelle nach Einbringen der Kern-DNA soll eine verbesserte Reprogrammierung der eingebrachten Kern-DNA erreicht werden.

Die WO 97/0669 betrifft ebenfalls ein Verfahren zur Wiederherstellung eines tierischen Embryos, bei dem der Kern einer "quiescent" (ruhenden) Donorzelle in eine geeignete Empfängerzelle transferiert wird. Gemäß dieser Druckschrift wird es als erforderlich angesehen, daß die Donorzelle vor Vereinigung mit der Empfängerzelle in der G0-Phase festgesetzt wird, was durch Hungern der Zellen oder Kontaktinhibition erreicht werden kann.

Schnieke et. al. (Science 278, 2130-2133, 1997) berichten über humane Faktor-IX transgene Schafe, die durch den Kerntransfer transformierter fetaler Fibroblasten erzeugt wurden. Dazu wurden geklonte Transfektanten als Spender für den Kerntransfer auf enukleierte Eizellen verwendet. Das genomische Konstrukt für den humanen Koagulationsfaktor IX war derart konstruiert, dass es in der Schafsmilch exprimiert wurde.

Wilmut et al. (Nature, 385, 810-813, 1997) beschreiben die Verwendung ruhender differenzierter Zellen für den Kemtransfer in eine geeignete Empfängerzelle. Implantation in ein Muttertier führte zur Geburt eines lebenden Lamms, was darauf hinwies, dass die Differenzierung von Zellen keine irreversiblen Veränderungen des genetischen Materials zu Folge hat, das zur Entwicklung erforderlich ist.

Cibelli et al. (Science, 280, 1256-1258, 1998) berichten die Erzeugung klonierter transgener Kälber aus nicht ruhenden "non-quiescent" fetalen Fibroblasten. Dazu wurden sich aktiv teilende fetale Fibroblasten genetisch verändert und mit enukleierten reifen Eizellen fusioniert. Beim Transfer von 28 Embryonen auf 11 Empfängerkühe wurden 3 gesunde, identische transgene Kälber erzeugt.

In der WO 98/30683 wird ein Verfahren beschrieben, das Kerne differenzierter Spenderzellen in enukleierte Eizellen bereitstellt. Die sich daraus ergebenden Kerntransfereinheiten werden zur Vermehrung von Genotypen und transgenen Genotypen durch Erzeugen von Feten und Nachkommen und zur Erzeugung isogener CICM-Zellen, einschließlich humaner isogener embryonaler Zellen oder Stammzellen, eingesetzt.

Die WO 99/01163 beschreibt ein Verfahren, das Kerne nicht serumgehungerter Spenderzellen nutzt, um sie in enukleierte Eizellen der gleichen Art einzubringen. Die sich daraus ergebenden Kerntransfereinheiten werden zur Vermehrung von Genotypen und transgenen Genotypen durch Erzeugen von Föten und Nachkommen und zur Erzeugung isogener CICM-Zellen, einschließlich humaner isogener embryonaler Zellen oder Stammzellen, verwendet.

Ein Problem bei dieser Technologie besteht jedoch immer noch darin, geeignete Spender-Zellen für den Kerntransfer zu finden, mit der sich ein tierischer Embryo am zweckmäßigsten und wirtschaftlichsten herstellen läßt. Bekanntermaßen stellt die Reprogrammierung der Kern-DNA aus der jeweils gewählten Spenderzelle die größte Schwierigkeit bei der Embryoklonierung dar, da diese nicht nur Einfluß hat auf die weitere frühe Reifung des Embryos, sondern auch auf die spätere Entwicklung nach einer gegebenenfalls durchgeführten Einpflanzung in ein Muttertier. So bestehen trotz aller Erfolge auf diesem Gebiet immer noch Probleme hinsichtlich einer effektiven Reprogrammierung der Spender-Kern-DNA, um die manipulierte Eizelle mit neuem Kern dem Zustand einer natürlichen Zygote anzunähern. Dies drückt sich u.a. in einer äußerst geringen Ausbeute hinsichtlich der Gewinnung embryonaler Blastozysten und einer geringen Teilungsrate aus.

Eine Aufgabe der vorliegenden Erfindung besteht daher darin, die Nachteile des Standes der Technik zu überwinden und eine geeignete Kern-Spenderzelle zur Verfügung zu stellen, mit der ein verbessertes Verfahren zur Herstellung von Ungulatenembryonen bereitgestellt werden kann.

Diese Aufgabe wird gelöst durch ein Verfahren zur Herstellung von Ungulatenembryosen, bei dem als Spenderzelle für den Kerntransfer fetale Fibroblasten eines Ungulaten aus einer sub-konfluenten Primärkultur eingesetzt werden. Der Kern dieser Zelle wird mit einer geeigneten Empfängerzelle zusammengebracht wobei der fetale Fibroblast vor der Vereinigung nicht durch externe Manipulation in der G0 Phase festgesetzt wird, um einen Karyoplast-Zytoplast-Komplex (KZK) zu erzeugen. Die Aktivierung des KZK erfolgt i) durch Inkubation des KZK für 5 Minuten in 7%igen Ethanol, ii) Inkubation des KZK aus i) für 5 Stunden in 10µg/ml Cycloheximid und 5µg/ml Cytochalasin B, und die so erhaltene Zelle wird für einen Zeitraum gezüchtet, damit sich eine Morula bildet. Die daraus erhältliche Morula kann dann gegebenenfalls zum Klonieren wiederverwendet werden.

Gemäß einer bevorzugten Ausführungsform der vorliegenden Erfindung ist die Empfängerzelle eine enukleierte Eizelle, in die der Kern des fetalen Fibroblasten zum Kerntransfer eingebracht werden kann oder mit der der fetale Fibroblast selbst fusioniert wird.

Die Tiere, bei denen das erfindungsgemäße Verfahren durchgeführt werden kann, sind Ungulaten, insbesondere Schweine, Schafe, Ziegen, Rinder bzw. Kühe.

In einer bevorzugten Ausführungsform sind die in dem Verfahren eingesetzten fetalen Fibroblasten transgen, d.h. sie enthalten ein oder mehrere Gene, die entweder von einer exogenen Quelle abgeleitet sind oder die ein endogenes, an einen anderen, nicht natürlichen Locus im Genom eingebrachtes Gen darstellen. Diese Gene codieren vorzugsweise für ein Arzneimittel, beispielsweise einen Antikörper oder einen ernährungsphysiologisch interessanten Stoff, beispielsweise Chymosin oder Trypsin, wobei die Gene jeweils unter der Kontrolle eines oder des endogenen oder eines exogenen Promotors liegen können.

Zur Gewinnung fetaler Fibroblasten werden Feten aus graviden Ungulaten gewonnen, beispielsweise durch einfaches Zerkleinern des Fetus. Die aus dem Fetus gewonnen Zellen werden sodann auf die gewünschten fetalen Fibroblasten selektiert, wie beispielsweise durch Anhaftenlassen an das Kulturgefäß und Abtrennen des Überstandes oder durch mechanische Selektion mittels einer Pipette. Fetale Fibroblasten lassen sich aufgrund ihres Phänotyps von anderen Zellen leicht unterscheiden.

Für die nachfolgenden Schritte kann der gewonnene fetale Fibroblast als solcher eingesetzt werden, oder der Kern kann daraus isoliert und weiterverwendet werden.

Als Empfängerzellen werden in der Regel enukleierte Eizellen eingesetzt, die in vivo oder in vitro gereift sind. So können beispielsweise in vitro gereifte, unbefruchtete Eizellen eingesetzt werden, bei denen nach Erreichen der Metaphase II die umgebenden Cumuluszellen entfernt wurden.

Die Empfängerzelle soll vorzugsweise keine eigene Kern-DNA aufweisen. Für die Entfernung der Eizell-DNA sind im Stand der Technik mehrere Möglichkeiten vorhanden, wie beispielsweise die Trennung der Eizelle in zwei Hälften, von denen eine Hälfte keinen Kern mehr aufweist und weiterverwendet werden kann, oder eine Bestrahlung mit ultraviolettem Licht zur Zerstörung der zelleigenen DNA. Eine Entfernung des Kerns bzw. der Pro-Nuclei oder der Metaphasen-Platte mittels Mikromanipulation ist ebenfalls möglich. Als bevorzugt hat sich eine Behandlung der Eizellen vor der Mikromanipulation mit Cytochalasin B mit anschließendem Absaugen des in der Nähe des Polkörpers liegenden Zytoplasmas mit Hilfe einer Pipette, beispielsweise mit einem Leitz-Micromanipulator (Leica, Bensheim, Deutschland) geführt, erwiesen. Da die Kern-DNA der Eizelle zu diesem Zeitpunkt in der Nähe der Polkörperchen lokalisiert ist, ist die Enukleationsrate bei dieser Methode sehr hoch, wobei gleichzeitig nur ein kleiner Teil des Cytoplasmas mit abgesaugt wird.

Nach Gewinnen der jeweils beim Kerntransfer beteiligten Zellen können generell zwei Wege beschritten werden. Der Kern des fetalen Fibroblasten wird anhand im Stand der Technik bekannter und etablierter Verfahren isoliert und in die vorbereitete Empfängerzelle eingebracht, wie beispielsweise mittels Injektion oder der fetale Fibroblast selbst wird mit der Empfängerzelle fusioniert.

Bei einer Fusion kann ein fetaler Fibroblast mit Hilfe einer geeigneten Vorrichtung, wie einer Transferpipette unter die Zona pellucida der enukleierten Eizelle geschoben und dort abgesetzt werden. Zur Integration des Zellkerns des fetalen Fibroblasten in das Zellplasma der Eizelle wird die Membran der Fibroblast mit der Membran der Eizelle fusioniert. Techniken zur Fusion von Zellen sind im Stand der Technik wohlbekannt, beispielsweise Fusion unter Verwendung des Sendai-Viruses, Behandlung mit PEG (Polyethylenglycol), Laserfusion oder Elektroschock. Die letztgenannte Methode, die sogenannte Elektrofusion, bei der durch gegebenenfalls mehrmalige, beispielsweise 2 bis 10 mal, kurzzeitige Gleichstrompulse von etwa 1 bis 5 kV/cm, vorzugsweise 1 bis 3 kV/cm, mit einer jeweiligen Dauer von 2 µsek. bis 1 sek., Poren induziert werden, die ein Zusammenfließen des Zytoplasmas ermöglichen, ist in dem vorliegenden Verfahren bevorzugt, da die elektrischen Pulse bei geeigneter Stärke gleichzeitig eine Aktivierung der (fusionierten) Eizelle mit sich bringen können. Die Aktivierung kann auch einige Stunden (ca. 2-5 Std.) nach der Fusion erfolgen, beispielsweise durch eine Inkubation der fusionierten Zelle in einer 7 %igen Alkohollösung, vorzugsweise einer 7 %igen Ethanollösung, oder anhand anderer im Stand der Technik bekannten Verfahren.

Die Aktivierung der fusionierten Zelle ist ein wichtiger Schritt, da sie die Voraussetzung für das Ingangkommen der Teilungsaktivität des Fusionsproduktes ist. Nach erfolgter Fusion und Aktivierung werden die Fibroblasten-Eizellenkomplexe (Kerntransferembryonen) gezüchtet, bis sie ein Stadium erreichen, in dem sie gegebenenfalls auf einen Empfänger transferiert werden können. Dabei können dem verwendeten Kulturmedium nach Wahl Stoffe zugesetzt werden, die die Aggregation von Mikrotubuli unterstützen oder inhibieren. Nocadazol sowie Colcemid sind Beispiele für die Aggregation inhibierende Mittel, Taxol ist ein Mikrotubuli-Stabilisator. Diese Stoffe verhindern eine gegebenenfalls auftretende Bildung mehrerer Pro-Nuclei.

Bei den bestehenden Verfahren des Standes der Technik mußten zur weiteren Züchtung der sich bildenden Embryonen diese sorgfältig in einen Zwischenempfänger überführt werden. Dies wurde im allgemeinen dadurch erreicht, daß der Embryo in einem schützenden Medium, wie Agar, verpackt in die Eileiter eines "einstweiligen Muttertiers" (temporärer Empfänger) überführt wurde, in dem eine weitere Entwicklung bis zur Einpflanzung in das (endgültige) Muttertier erforderlich war. Bei dem erfindungsgemäßen Verfahren ist es jedoch auch möglich bestehende in-vitro-Systeme für die Kultivierung einzusetzen ohne dabei die Ausbeuten zu verschlechtern. Ohne an eine Theorie gebunden zu sein, könnte diese Tatsache mit der getroffenen Auswahl der Spenderzelle einhergehen, mit der Embryonen erhalten werden können, die hinsichtlich Ihrer Entwicklung natürlich erzeugten Embryonen sehr nahe kommen. Die Zellen werden für einen Zeitraum in Kultur gehalten, bis sich Blastozysten bilden. Dies umfaßt einen Zeitraum von bis zu 10 Tagen, vorzugsweise 6 bis 7 Tagen.

Die fetalen Fibroblasten zum Einsatz in dem erfindurigsgemäßen Verfahren können aus einer Vielzahl von Ungulaten gewonnen werden wie beispielsweise Rinder, Schafe, Ziegen, Büffel, Kamele sowie Schweine. Am meisten bevorzugt sind Schafe bzw. Kühe.

Um die Isolierung des Genprodukts zu erleichtern kann das Genprodukt in ein Produkt des Tieres selbst gerichtet werden, bei Kühen bzw. Schafen beispielsweise in die Milch Dies kann durch Wahl geeigneter Promotoren zur organspezifischen Expression erreicht werden, die im Stand der Technik bekannt sind. Das Genprodukt kann jedoch gleichermaßen aus dem Tier selbst gewonnen werden, beispielsweise aus dem Serum. Auch ist es möglich, daß das/die Organ(e)/Gewebe der Tiere das gewünschte Produkt darstellen, wobei, beispielsweise für eine (Xeno-)Transplantation.

Die in dem erfindungsgemäßen Verfahren eingesetzten fetalen Fibroblasten bzw. die Feten oder Tiere, von denen sie abgeleitet sind, können darüber hinaus transgen sein, wobei das Transgen vorzugsweise für ein ernährungsphysiologisch oder pharmazeutisch interessantes Produkt, beispielsweise einen Antikörper codiert. Beispielsweise können bei Kühen oder Schafen die Gene für Chymosin oder Trypsin in ein Konstrukt eingebaut werden, das die Produktion des entsprechenden Enzyms, bzw. eines seiner Vorläufer in der Milch des Tieres ermöglicht. Das Transgen von Interesse kann dabei, je nach Wunsch, unter der Steuerung eines exogenen, ebenfalls transgenen Promotors liegen, oder ein bekannter endogener Promotor kann für diesen Zweck eingesetzt werden.

Eine ganze Reihe von Proteinen wird bislang aus tierischen Organen durch Aufreinigung aus diesen Organen gewonnen und dann in der Medizin oder Technik eingesetzt. Probleme dabei bestehen u.a. hinsichtlich der relativen Mengen, in denen sie in diesen Geweben vorhanden sind (beispielsweise FSH aus Hypophysen), was zu hohen Produktionskosten führt, da zur Gewinnung einerseits eine große Menge an Ausgangsmaterial, d.h. viele Tiere, erforderlich sind, was aufgrund der Vielzahl von Tieren die Gefahr einer Kontamination, mit beispielsweise Krankheitserregern, wie BSE oder Ehec, mit sich bringt.

Beispiele für interessierende Proteine aus Tierorganen sind Aprotinin aus der Lunge, Chymosin aus dem Magen, Katalase aus der Leber, Elastase, Pankreatin, Insulin oder Trypsin aus dem Pankreas, Hyaluronidase aus Hoden, Chondroitin aus Trachea, Kollagene aus der Haut, Fibronectin oder Vitronectin aus Plasma, Epithelial Cell Growth suppl. oder LH (Luteinisierendes Hormon) aus der Hypophyse, Fibroblast growth factor oder Ganglioside aus dem Hirn, sowie Hämoglobin, Thrombin, Transferrin usw.. Diese Aufzählung ist nicht als Einschränkung anzusehen.

Für alle diese Produkte kann eine ektotope Expression, d.h. eine Expression in einem anderen Gewebe, beispielsweise in der Milchdrüse erreicht werden, wenn in den für die Klonierung verwendeten Zellen vorher entweder ein additiver Gentransfer durchgeführt wurde, beispielsweise über Injektion, Transformation, Transfektion oder anhand eines anderen im Stand der Technik bekannten Verfahrens, wobei ein in vitro rekombiniertes Genkonstrukt zusätzlich ins Genom integriert wird. Darüber hinaus kann durch homologe Rekombination in den Zellen erreicht werden, daß das endogen vorhandene Gen mit einem Promotor gekoppelt wird, der für dieses Strukturgen ein anderes Expressionsmuster ergibt, beispielsweise Produktion von Chymosin im Euter mit einhergehender Sezernierung in die Milch anstelle der endogenen Synthese im Magen. Weiter kann ein endogen vorhandener Promotor, beispielsweise der Casein- oder Lactoglobulin-Promotor mit einem neuen Strukturgen gekoppelt werden, so daß für die Expression optimale Bedingungen vorliegen. In beiden vorstehend aufgeführten Fällen können Promotor und Strukturgen, die homolog in das Genom hinein rekombiniert werden, vorab aus einer Genbank isoliert werden, die beispielsweise aus fetalen Fibroblasten gewonnen worden ist, so daß nicht nur spezieseigene DNA (Selbstklonierung) eingesetzt wird, sondern es sich auch um isogene DNA handelt.

Auf diesem Weg kann daher die Zusammensetzung von Nahrungsmitteln, die aus tierischen Produkten, wie beispielsweise Milch, gewonnen werden, in gewünschter Weise verändert werden, so daß diese positive alimentäre, dietätische, gesundheitsfördernde Eigenschaften oder ein geringeres allergenes Potential, bessere Lagerungsstabilität oder Verarbeitungseigenschaften aufweisen. So kann beispielsweise Milch mit Ehec-Antikörpern oder Milch mit speziell auf Erkrankungen, wie beispielsweise Leaktoseintoleranz, abgestimmten Eigenschaften hergestellt werden.

Darüber hinaus können durch Verwendung von MACs (Mammalian artificial Chromosomes) Integrationsmutationen vermieden und große DNA-Fragmente transferiert werden. Dieses MACs werden als zusätzliche Mini- bzw. Mikrochromosomen im Kern genauso repliziert, wie die endogenen Chromosomen. Dadurch ist es beispielsweise möglich, über die Spezies hinweg Gencluster zu transferieren, beispielsweise komplette Immunglobulin-Gencluster des Menschen auf Nutztiere. wobei dieses Nutztier dann in der Lage wäre, humane Antikörper zu produzieren, die gewonnen und genutzt werden könnten. Der Transfer bestimmter MACs aus der eigenen Spezies führt weiter dazu, daß bei additiven Geneffekten eine Erhöhung der Synthese des Genprodukts folgern würde.

Wichtig ist auch eine Expression homologer Proteine, bzw. auch Geweben oder Organen in Nutztieren, bei Proteinen in den gleichen Organen, in denen diese Proteine auch beim Menschen exprimiert werden. Die Proteine werden dann anhand im Stand der Technik bekannter Verfahren gewonnen, die Gewebe bzw. Organe vor einer eventuellen Transplantation aus dem Tier entnommen. Der Vorteil dieser Vorgehensweise besteht in einer hohen Identität der exprimierten Proteine, da sie im richtigen Organ prozessiert bzw. post-translational modifiziert werden, beispielsweise Expression von Erythropoietin in der Niere. Die führt dazu, daß die aus den unterschiedlichen Geweben gewonnenen Proteine die gleiche Glycosylierung aufweisen, wie die Stoffe im Menschen selbst, wobei deren Aktivität der des natürlichen Proteins sehr nahe kommt. So können transgene Tiere, beispielsweise Schweine, Rinder usw. erhalten werden, die menschliches Insulin, Erythropoietin usw. produzieren, die in der Medizin dann besser genutzt werden können.

Ein Vorteil des erfindungsgemäßen Verfahrens ist neben Umgehung des Erfordernis die Zellen in die G0 Phase zu bringen auch eine gleichbleibende und sogar gesteigerte Effizienz hinsichtlich der Ausbeuten bei der Reklonierung.

So konnten unter Verwendung des erfindungsgemäßen Verfahrens Ergebnisse erzielt werden, die sogar besser waren als jene, die aus punktierten Oozyten mit anschließender Reifung und Fertilisation - jedoch ohne Klonierung - entstanden sind. Die dabei beobachtete höhere in vivo Entwicklungskapazität erhöht die Effizienz der Klonierungsprogramme erheblich.

Als Maß für die Effizienz derartiger Verfahren kann die sogenannte Graviditätsrate (Trächtigkeitsrate) dienen, die als Anteil der nach Transfer von 6 - 7 Tagen in vitro kultivierten Embryonen auf synchronisierte Empfängertiere gravid gewordenen Tiere ermittelt werden kann. Die jeweiligen Graviditätsraten können durch Messung des Progesteronspiegels, Ultraschalluntersuchungen oder rnittels rektaler Palpation bestimmt werden.

Dabei wurden beim Beispiel Rind mit unterschiedlichen Verfahren folgende Ergebnisse erhalten:

| Graviditätsraten: | |
|---|---|
| Oocytenpunktion mit anschließender IVM und IVF | 34 % |
| Embryoklonierung (durchschnittlich) | 25 % |
| Embryoklonierung mit fetalen Fibroblasten | 55 % |
| IVM = in vitro Maturation/Reifung | |
| IVF = in vitro Fertilisation | |

Die Erfindung wird nun unter Bezugnahme auf das lediglich zur Erläuterung gegebene Beispiel ausführlicher erläutert, das den Bereich der Erfindung nicht einschränken soll.

### Beispiel

### Isolierung fetaler Fibroblasten beim Rinderfetus

Feten aus Uteri von geschlachteten Kalbinnen oder Kühen wurden freipräpariert und in PBS (Phosphate buffered Saline, ohne Ca²⁺/Mg²⁺, mit Penicillin/ Streptomycin, plus 10% fetales Kälberserum (FCS)) auf Eiswasser ins Labor gebracht. Die Feten wurden anschließend mehrmals mit frischem PBS gewaschen. Nach dem Waschen wurden die Feten vom Kopf und den inneren Organen befreit, nochmals in PBS gewaschen, in 5 ml PBS zerkleinert und in ein 50 ml Kulturröhrchen überführt. Nach Zugabe von 10 ml PBS wurde bei 300 Upm für 5 min vorsichtig zentrifugiert und das Pellet in 0,1 %iger Trypsinlösung resuspendiert. Nach einer 5-minütigen Inkubation bei 37°C erfolgte ein Transfer in ein 50 ml Zentrifugenröhrchen, worauf erneut zentrifugiert wurde (300 Upm/5 min.). *Diese* Schritte, beginnend mit der Trypsinbehandlung wurden zweimal wiederholt. Die so erhaltene Zellsuspension wurde dann filtriert, in ein 50 ml Röhrchen übernommen, für 5 min. zentrifugiert (160 g), das Pellet resuspendiert und in 1 ml Kulturmedium (Dulbecco's modifiziertes Eagle Medium (Gibco), ergänzt mit 15% FCS, 2mM L-Glutamin, 10⁻⁷ mMol β-Mercaptoethanol, und Penicillin/Streptomycin) aufgenommen.

Nach der Trypsinbehandlung wurde die Zellsuspension in 10 cm Kulturschalen gegeben und in Kulturmedium (supra) mit 10 % FCS (Biochrom, Berlin) solange gezüchtet (37°C, 5 % CO₂), bis der Zellrasen subkonfluent war (2 bis 3 Tage). Ein Teil dieser Passage "0" wurde eingefroren (10 % Dimethylsulfoxid, Sigma) und in flüssigem Stickstoff gelagert.

Als Vergleich hinsichtlich der Effizienz des Verfahrens wurde ein Teil der Fibroblasten vor dem Kerntransfer nach dem in der WO 97/07669 (Campell et al.) beschriebenen Verfahren durch starke Verringerung der Serumkonzentration in der Kultur in eine presumptive G0-Phase synchronisiert. Dazu wurden Zellen einen Tag nach der Passage dreimal mit PBS gewaschen und dann in frischem Medium mit 0,5 % FCS für 8 Tage gezüchtet (Starvation, "gehungerte Zellen"), bevor sie für die Klonierung verwendet wurden. In dem von Campell beschriebenen Verfahren wird es als unumgänglich angesehen, daß die eingesetzten Fibroblasten durch "Hungern" oder andere Verfahren in die G0-Phase überführt werden.

Die Fibroblasten für das hier beschriebene Verfahren, die diesem Aushungerungsprozeß (Starvation) nicht ausgesetzt wurden, wurden direkt aus der subkonfluenten Zellkultur entnommen und ohne weitere Behandlung für die Klonierung verwendet.

Weiter wurden Blastomeren (Embryonalzellen) aus Morulae (Embryonen im Alter von etwa 6 Tagen mit einer Zellzahl zwischen 30 und 70 Blastomeren), die aus der Klonierung mit gehungerten und nicht behandelten Fibroblasten entstanden sind, erneut für die Klonierung verwendet (Reklonierung). Die Ergebnisse sind in der Tabelle zusammengestellt und zeigen, daß trotz Arbeiten gegen die Lehre des Standes der Technik sogar bessere Ergebnisse erhalten werden können.

Auch bei den erhaltenen Graviditätsraten konnte überraschenderweise festgestellt werden, daß diese bei 60 % lag.

### Additiver Gentransfer

In vitro rekombinierte Genkonstrukte, wie sie in der DE-OS-40 12 526 beschrieben sind, die hier unter Bezugnahme mit aufgenommen wird, werden durch konventionelle DNA-Mikroinjektion (Brem G., Transgenic Animals, Genetic Engineering of Animals, VCH Weinheim (1993), 83-170) in die Kerne isolierter fetaler Fibroblasten oder durch bekannte Transformationsverfahren (Maniatis et al., Molecular Cloning. A Laboratory Manual, Cold Spring Harbor Laboratory, 1989) stabil integriert. Der Nachweis der Integration in den Zellen erfolgt mittels PCR und/oder Southern Blotting (Maniatis, vorstehend). Eine Expression in ausdifferenzierten Zellen zeigt, daß der Gentransfer erfolgreich gewesen ist.

### Klonierung

18-20 Std. nach dem Beginn der Reifung wurden aus dem Eierstock isolierte bovine Oozyten von den sie umgebenden Kumuluszellen befreit und innerhalb von zwei Stunden enukleiert (Molecular Reproduction and Development **42** (1995), 53-57). Ca. 20-22 Stunden nach Beginn der Reifung wurden wie vorstehend gewonnene fetale Fibroblasten mittels einer Transferpipette in den perivitellinen Raum von enukleierten Oozyten überführt und die sich dabei bildenden Karyoplast-Zytoplast-Komplexe (KZK) wurden jeweils für 10 µsek. einem doppelten elektrischen Puls von 2,1 kV/cm ausgesetzt, um die Fusion zu induzieren. Die KZKs wurden in Ham's F-12 Medium (Sigma) mit 10% FCS in einem Brutschrank gezüchtet. Die Fusion wurde 30 bis 60 Minuten nach dem Fusionspuls durch mikroskopische Untersuchung beurteilt.

24 Stunden nach Beginn der Reifung wurden die KZKs durch eine 5 minütige Inkubation in 7% Ethanol aktiviert und anschließend 5 Stunden in 10µg/ml Cycloheximid (Sigma C-7698) und 5 µg/ml Cytochalasin B (Sigma C-6762) gezüchtet. Anschließend wurden die KZKs in einem 100 µl Tropfen CR-1 Medium (Rosenkrans und First, 1991) mit 10% Östrus-Kuh-Serum umgesetzt. Die Tropfen wurden mit Paraffin-Öl überschichtet und für 7 bis 8 Tage bei 39°C in wasserdampfgesättigter Atmosphäre aus 5% CO₂, 5% O₂ und 90 % N₂ gezüchtet.

**Tabelle 1:**

| Morulae aus fetaler Fibroblastenklonierung (FFB) als Kernspender | | | | |
|---|---|---|---|---|
| Kerntransfer-Morulae aus Klonierung | KZK | Fusionierte FFB (%) | Teilung (%) | Blastozysten (%) |
| FFB (nicht gehungert) | 65 | 58 (89%) | 50 (86%) | 32 (55%) |
| FFB (gehungert) | 102 | 91 (88%) | 73 (80%) | 47 (52%) |

Wie aus vorstehender Tabelle ersichtlich, war es bereits bei dem ersten Versuch möglich, eine Teilungsrate bis zu 86 % und eine Blastozystenrate von bis zu 55 % zu erhalten. Dies ist im Hinblick darauf, daß es im Stand der Technik als erforderlich angesehen wird die Fibroblasten "auszuhungern", als überraschend anzusehen, da Ergebnisse erzielt werden können, die gegenüber dem Stand der Technik sogar besser sind.

### Embryotransfer

### Empfängermanagement

Als Empfänger wurden Kalbinnen verwendet, die folgende Kriterien erfüllten:
1. Aufzucht in IBR (bovines Herpesvirus Typ 1) unverdächtigen Betrieben;
2. serologische Untersuchung auf BHV-1-Antikörper (infektiöse bovine Rhinotracheitis /infektiöse pustulöse Vulvovaginitis) negativ;
3. serologische Untersuchung auf BVD (bovine Virus Diarrhoe)/MD-Antigen (Mucosal Disease) negativ;
4. dem Alter (13-16 Monate) entsprechende Körpermasseentwicklung;
5. eingetretene Geschlechtsreife; bei Tieren, die den Embryo austragen sollen, eingetretene Zuchtreife;
6. gynäkologische Untersuchung ohne pathologische Befunde;

Alle Empfänger erhielten unmittelbar nach Aufstallung Mineralstoffboli (All Trace, Ranching Consult GmbH), um die erfahrungsgemäß unzureichende Versorgung mit. Selen, Kupfer und Cobalt auszugleichen (Wittkowski et al., Zur Selensupplementierung bei Färsen; Jahrestagung der Arbeitsgemeinschaft Embryotransfer Deutschland (AET-d). 13.06.-14.06.1996, Marktredwitz).

BVD-Antikörper negative Tiere werden gegen BVD/MD immunisiert (Rumilis®, Intervet), um das Infektionsrisiko bei Übertragung von Embryonen (Mödl et al., Control of bovine viral diarrhea virus in abattoir ovaries for in vitro fertilization (IVF) or cloning programs; 11e Reunion A.E.T.E.-Hannover, 8-9 September 1995) bzw. nach Verbringen in Stallungen mit unbekanntem BVD-Status zu minimieren. Die Fütterung erfolgte ad libitum mit Grassilage, Heu und Stroh. Entwurmungen wurden im Frühjahr und Herbst mit Ivermectin (Ivomec®, MSD Agvet) durchgeführt. Die Unterbringung der Empfänger erfolgt zum Teil in Laufstall- (Offenstall, Gruppengröße 6 Tiere) und zum Teil in Anbindehaltung.

### Empfängervorbereitung

Die Übertragung der in vitro hergestellten Embryonen erfolgt auf Zyklus-synchrone Empfänger, d.h. das Stadium des Sexualzyklus entspricht dem Alter der zu übertragenden Embryonen. Dabei wird der Tag der Brunst als Zyklustag 0 bezeichnet. Die Brunstsynchronisation erfolgt im Diöstrus durch die einmalige intramuskuläre Applikation eines Prostaglandin F₂-α-Analogons (2,0 ml Estrumate®, Mallinckrodt Veterinary). Kalbinnen, bei denen mittels rektaler Palpation kein funktionelles Corpus luteum diagnostizierbar war, wurden nicht für die Brunstsynchronisation verwendet. Die Brunst tritt erfahrungsgemäß 2-3 Tage post applicationem ein und wird anhand des Brunstverhaltens und des Scheidenbefundes beurteilt.

### Embryotransfer

Die in vitro produzierten Embryonen wurden nach 7-tägiger Kultur auf geeignete Empfänger transferiert. Dazu wurden die Embryonen identifiziert, qualitativ beurteilt, Zona geschlitzt, in ein geeignetes Transfermedium umgesetzt und anschließend in Minipailletten (Minitüb) aufgezogen. Als Transfermedien wurden PBS + 10% fetales Kälberserum (FCS, Biochrom), Ovum Culture Medium (ICP, Neuseeland) + 10% FCS oder TL-Hepes + 10% FCS verwendet.

Die verschlossenen Pailletten wurden bis zum Transfer, der innerhalb von ca. 90 Minuten stattfinden sollte, bei 37,8°C in einem Miniinkubator gelagert.

Die Eignung der Empfänger wurde anhand folgender Kriterien beurteilt:

Die Tiere wurden etwa 7 Tage vor dem Transfer in Brunst beobachtet, wobei die Asynchronität 24 Std. nicht überschreiten soll (Hasler et al., Theriogenology **43** (1995), 141-152). Das Vorhandensein und die Größe eines funktionellen Gelbkörpers wurde entsprechend bewertet (Assey et al., Theriogenology **39** (1993), 1321-1330).

Die verwendeten Tiere wiesen keine Anzeichen einer Erkrankung des Genitaltraktes auf.

Nach der Auswahl wurde eine Epiduralanästhesie (2,0 ml Lidocain®, Albrecht) vorgenommen und das äußere Genitale sorgfältig mit trockenem Papier gereinigt. Anschließend wurde der körperwarme Transferkatheter (Minitüb) mit einer Paillette beschickt und mit einer Plastikschutzhülle (Sanisheath, Minitüb) versehen. Der Transfer erfolgte unblutig unter rektaler Kontrolle der Cervixpassage und der Katheterposition in die Spitze des ipsilateralen Uterushornes (Reichenbach et al., J. Reprod. Fertil. **95** (1992), 363-370). Dabei wurde die Plastikschutzhülle erst am äußeren Muttermund mit dem Transferkatheter perforiert, um eine Keimverschleppung aus der Vagina in den Uterus zu vermeiden. War der Transfer mehrerer Embryonen auf einen Empfänger vorgesehen, so wurden diese bilateral abgesetzt. Dazu wurde der Transferkatheter bis in das Corpus uteri zurückgezogen, der Mandrin mit der entleerten Paillette entfernt, eine neue Paillette mit Embryo(nen) in den Katheter geschoben und im contralateralen Uterushorn positioniert. Unmittelbar nach dem Transfer wurden alle relevanten Daten (Lebensnummer des Empfängers, Herkunft, Anzahl und Qualität der Embryonen usw.) dokumentiert.

### Trächtigkeitsuntersuchung

21 Tage nach der Brunst, also 14 Tage nach dem Embryotransfer, wurde eine Brunstkontrolle vorgenommen und der Progesterongehalt im Blutserum festgestellt. Werte unter 0,1 ng/ml werden als nicht trächtig angesehen. Bei Progesteronwerten über 2,0 ng/ml kann mit einer Trächtigkeit gerechnet werden. Die erste direkte Trächtigkeitsuntersuchung wurde um den 35. Tag mittels Ultraschall und die zweite manuell um den 42. Trächtigkeitstag vorgenommen.

## Patentansprüche

1. Verfahren zur Herstellung von Ungulatenembryonen, das die folgenden Schritte umfasst,
a) Gewinnen eines fetalen Fibroblasten eines Ungulaten aus einer sub-konfluenten Primärkultur,
b) Vereinen des Kerns des fetalen Fibroblasten mit einer geeigneten Empfängerzelle, wobei der fetale Fibroblast vor der Vereinigung nicht durch externe Manipulation in der G0 Phase festgesetzt wird, um einen Karyoplast-Zytoplast-Komplex (KZK) zu erzeugen;
c) Aktivierung des KZK durch
i) Inkubation des KZK für 5 Minuten in 7%-igen Ethanol,
ii) Inkubation des KZK aus Schritt c)(i) für 5 Stunden in 10µg/ml Cycloheximid und 5µg/ml Cytochalasin B und
d) Züchten der so erhaltenen Zelle in-vitro für einen Zeitraum, damit sich eine Blastozyste bildet.

2. Verfahren nach Anspruch 1, bei dem die geeignete Empfängerzelle ein enukleierte Eizelle ist.

3. Verfahren nach einem der vorhergehenden Ansprüche, bei dem der fetale Fibroblast mit der geeigneten Empfängemelle fusioniert wird.

4. Verfahren nach einem der vorhergehenden Ansprüche, bei dem der Ungulat ein Rind ist.

5. Verfahren nach einem der vorhergehenden Ansprüche, bei dem der fetale Fibroblast transgen ist.

6. Verfahren nach Anspruch 5, bei dem der fetale Fibroblast ein pharmazeutisch oder ernährungsphysiologisch interessantes Gen enthält.

7. Verfahren nach Anspruch 5, bei dem das Transgen Chymitrypsin oder Trypsin ist.

8. Verfahren nach einem der Ansprüche 5 bis 7, bei dem das Transgen unter der Kontrolle eines endogenen Promotors liegt.

9. Verfahren nach einem der Ansprüche 5 bis 8, bei dem das Transgen oder ein endogenes Gen unter der Kontrolle eines exogenen Fremdpromotors oder einer Spezies endogenen Promotors liegt.

## Claims

1. A process for the production of ungulate embryos comprising the following steps:
a) collecting of a foetal fibroblast of an ungulate from a sub-confluent primary culture;
b) combining the nucleus of the foetal fibroblast with a suitable receiving cell, said foetal fibroblast not being halted by external manipulation in the G0-phase before said combination, to produce a karyoplast-cytoplast-complex (KCC),
c) activation of the KCC by
i) incubation of the KCC for 5 minutes in 7% ethanol;
ii) incubation of the KCC of step c)(i) for 5 hours in 10 µg/ml cycloheximide and 5 µg/ml cytochalasin B; and
d) culturing *in vitro* the cell thus obtained for a period such that a blastocyst is formed.

2. The process according to claim 1, wherein the suitable recipient cell is an enucleated oocyte.

3. The process according to any of the preceding claims, wherein the foetal fibroblast is fused with the suitable recipient cell.

4. The process according to any of the preceding claims, wherein the ungulate is bovine.

5. The process according to any of the preceding claims, wherein the foetal fibroblast is transgenic.

6. The process according to claim 5, wherein the foetal fibroblast contains a pharmaceutically or dietetically gene of interest.

7. The process according to claim 5, wherein the transgene is chymotrypsin or trypsin.

8. The process according to any of the claims 5 to 7, wherein the transgene is under the control of an endogenous promotor.

9. The process according to any of the preceding claims 5 to 8, wherein the transgene or an endogenous gene is under the control of an exogenous foreign promotor or of a species' endogenous promotor.

## Revendications

1. Procédé pour la fabrication d'embryons d'ongulés qui comprend les étapes suivantes :
a) obtention d'un fibroblaste foetal d'un ongulé à partir d'une culture primaire subconfluente ;
b) fusion du noyau du fibroblaste foetal avec une cellule réceptrice appropriée au cours de laquelle le fibroblaste foetal n'est pas fixé phase G 0 avant fusion par une manipulation externe, pour produire un complexe karyoplaste-cytoplaste;
c) activation du KZK par
i) incubation du KZK durant 5 minutes dans l'éthanol à 7 %,
ii) incubation du KZK de l'étape c) (i) durant 5 heures dans du cycloheximide à 10 µg/ml et de la cytochalasine à 5 µg/ml ; et
d) culture in vitro des cellules ainsi obtenues sur une période telle qu'il se forme un blastocyste.

2. Procédé selon la revendication 1 dans lequel la cellule réceptrice appropriée est un ovule énucléé.

3. Procédé selon l'une des revendications précédentes dans lequel le fibroblaste foetal est fusionné avec la cellule réceptrice appropriée.

4. Procédé selon l'une des revendications précédentes dans lequel l'ongulé est un bovidé.

5. Procédé selon l'une des revendications précédentes dans lequel le fibroblaste foetal est transgénique.

6. Procédé selon la revendication 5 dans lequel le fibroblaste foetal contient un gène intéressant du point de vue pharmaceutique ou physiologique nutritionnel.

7. Procédé selon la revendication 5 dans lequel le transgène est la chymotrypsine ou la trypsine.

8. Procédé selon l'une des revendications 5 à 7 dans lequel le transgène est sous le contrôle d'un promoteur endogène.

9. Procédé selon l'une des revendications 5 à 8 dans lequel le transgène, ou un gène endogène, est sous le contrôle d'un promoteur étranger exogène ou d'un promoteur d'espèce endogène.
